# EUROPEAN PATENT SPECIFICATION

(11) **EP 0 468 232 B1**
(45) Date of publication and mention of the grant of the patent: **03.02.1999**
(21) Application number: 91110933.8
(22) Date of filing: 02.07.1991
(51) Int. Cl.: A61K 47/32, A61K 9/10, A61K 9/08

(54) **Suspending syrups**
Suspendierfähige Sirupe
Sirops ayant des propriétés de mise en suspension

(30) Priority: 23.07.1990 JP 194443/90
(43) Date of publication of application: 29.01.1992
(73) Proprietor: SS PHARMACEUTICAL CO., LTD., Chuo-ku, Tokyo 103 (JP)
(72) Inventor: Otsuka, Shigenori, Chiba-shi, Chiba-ken 260 (JP); Kurazumi, Toshiaki, Narita-shi, Chiba-ken 286 (JP); Imamori, Katsumi, Yotsukaido-shi, Chiba-ken 284 (JP); Iwasa, Akira, Yotsukaido-shi, Chiba-ken 284 (JP)
(74) Representative: Wächtershäuser, Günter, Prof. Dr.

(56) References cited:
- EP-A- 0 293 885
- DE-A- 3 106 619
- FR-A- 2 115 409
- US-A- 3 346 449
- CHEMICAL ABSTRACTS, vol. 111, no. 2, 10 July 1989, Columbus, Ohio, US; abstract no. 12462A, A. BANGA ET AL.: 'Incorporation of simethicone into syrupy or clear liquid orals'

## Description

### BACKGROUND OF THE INVENTION

### 1) Field of the Invention

The present invention relates to suspending syrups containing a slightly water-soluble medicine, which remain in a well-dispersed state and retain good thermal stability and sufficient fluidity over a prolonged period of time and which are palatable.

### 2) Description of the Related Art

US-A-3,346,449, EP-A-0 293 885, FR-A-2 115 409 and DE-A-31 06 619 disclose liquid pharmaceutical preparations comprising Carbopol 934®.

Further compositions comprising Carbopol are known from Chem. Abstr., Vol. 111, no. 2, no. 12462 A, 1989.

A suspending syrup can be formulated as a pharmaceutical preparation where a medicine is either insoluble or only slightly soluble in water or a medicine gives an unpleasant taste and becomes barely palatable if formulated into a solution. This suspending syrup is required to remain in a well-dispersed state and also to retain good thermal stability and fluidity, because any prescribed dose of the medicine is determined by the quantity of the syrup to be taken and the syrup must be pleasant to take.

From such viewpoints, cellulose derivatives have heretofore been used as suspending syrups to achieve uniform dispersion of slightly water-soluble medicines, including microcrystalline cellulose · carboxymethyl cellulose sodium ("AVICEL"; trade name), carboxymethyl cellulose, sodium carboxymethyl cellulose, methylcellulose, hydroxypropylcellulose and hydroxypropyl methylcellulose.

To maintain a suspending syrup in a well-dispersed state, in other words, to prevent settling of suspended particles, the following methods have been known:
A. To reduce the size of the suspended particles.
B. To minimize the difference in density between the suspended particles and the dispersion medium.
C. To increase the viscosity of the dispersion medium.

Of these, method A or B can bring about some improvements when practiced by mechanically dividing the suspended particles or adding a sugar such as sucrose to control the specific gravity. Such improvements are however not fully satisfactory. Although higher viscosity can prevent settling, method C is accompanied by the drawback that higher viscosity naturally leads to poor fluidity thereby making it difficult to dispense the syrup into smaller containers and also to administer the syrup. To overcome this drawback, an attempt was made to provide a thixotropic suspending agent that would remain in the form of a gel of high viscosity during storage but, after being shaken, would change to a sol having fluidity. The suspension so produced was however accompanied by the drawbacks that the suspension easily solated under vibrations during transportation or the like, suspended particles became more susceptible to settling once solation had taken place, and the thermal stability of the suspension was not good.

### SUMMARY OF THE INVENTION

Accordingly, there has been a desire for the development of a suspending syrup which is free from the settling of suspended particles, remains in a stably-dispersed state and has good thermal stability and fluidity.

With the foregoing circumstances in view, the present inventors have proceeded with an extensive investigation. As a result, it has been found that use of carboxyvinyl polymer as a suspending agent can provide a suspending syrup which, even when stored over a prolonged period of time, retains a well-dispersed state without settling of particles dispersed therein and has good thermal stability and fluidity, leading to the completion of the present invention.

The present invention therefore provides a suspending syrup with a slightly water-soluble medicine selected from the group consisting of tranilast, mefenamic acid, fenbufen, tipepidine hibenzate, pranoprofen, erythromycin ethyl succinate and domperidone, comprising carboxyvinyl polymer and having a viscosity of from 100 to 1,000 cps at 20°C.

The present invention can provide excellent suspending syrups, each of which has substantially the same viscosity both when left over standstill and after shaken and, even at high temperatures, remains free from the settling of suspended particles and retains high stability and good fluidity, and moreover is easy to handle and pleasant to take.

### DETAILED DESCRIPTION OF THE INVENTION AND PREFERRED EMBODIMENTS

Carboxyvinyl polymer is incorporated as a suspending agent in the present invention. Commercial products can be used, e.g. "Carbopol" (trade name; including "Carbopol 934P", "Carbopol 934", "Carbopol 940", "Carbopol 941" and "Carbopol 910", all products of B.F. Goodrich Chemical Co.) and "HIVISWAKO" (trade name; including "HIVISWAKO 103", "HIVISWAKO 104" and "HIVISWAKO 105", all products of Wako Pure Chemical Industries, Ltd.). Although no particular limitation is imposed on the content of carboxyvinyl polymer as long as it is sufficient for the uniform dispersion of the slightly water-soluble medicine, the content of carboxyvinyl polymer may range from 0.1 g to 2.0 g per 100 mℓ with 0.1-1.0 g/100 mℓ being especially preferred.

The slightly water-soluble medicine usable in the present invention is selected from the group consisting of tranilast, mefenamic acid, fenbufen, tipepidine hibenzate, pranoprofen, erythromycin ethyl succinate and domperidone.

Other known suspending agents can also be incorporated in the suspending syrup of the present invention to extent not impairing the advantages of the present invention. Illustrative of such suspending agents are cellulose derivatives such as microcrystalline cellulose · carboxymethyl cellulose sodium, carboxymethyl cellulose, sodium carboxymethyl cellulose, methylcellulose, hydroxypropylcellulose and hydroxypropyl methylcellulose. It is also possible to incorporate ingredients generally contained in suspending syrups, for example, antiseptics such as sodium benzoate and butyl p-hydroxybenzoate, corrigents such as sucrose and sorbitol, and, as dispersing agents, surfactants such as polyoxyethylene hydrogenated castor oil, polysorbate 80 and sugar ester.

The suspending syrup according to the present invention can be prepared by stirring the above ingredients into an intimate mixture in a manner known *per se* in the art. Here, the viscosity of the suspending syrup is adjusted to 100-1,000 cps at 20°C from the standpoints of dispersion stability and palatability. The viscosity adjustment can be performed by adjusting the amounts of carboxyvinyl polymer and other dispersing agents to be added. It is preferable to adjust the pH of the suspending syrup to 3-6 with a pH adjuster.

The present invention will next be described in further detail by the following examples and comparative examples.

### Examples 1-3 and Comparative Examples 1-3

Suspending syrups of the compositions shown in Table 1 were prepared by procedures to be described below. Their viscosities were investigated while they were left over standstill and also after they were shaken. Also investigated were variations of settling of particles on standing. The results are summarized in Table 2.

### (Procedures)

In each of the examples and comparative examples, sodium benzoate and sucrose were dissolved in a suitable amount of purified water which had been heated. Where the composition contained microcrystalline cellulose · carboxymethyl cellulose sodium, the resultant solution was then added with the cellulose derivative, followed by dispersion under stirring in a homomixer. Added to the resulting solution was a dispersion of tranilast in an aqueous solution which contained an aqueous solution of carboxyvinyl polymer, said aqueous solution having been prepared separately, sodium carboxymethyl cellulose and polyoxyethylene hydrogenated castor oil. The resultant mixture was finally adjusted to pH 5.0 with citric acid or sodium citrate.

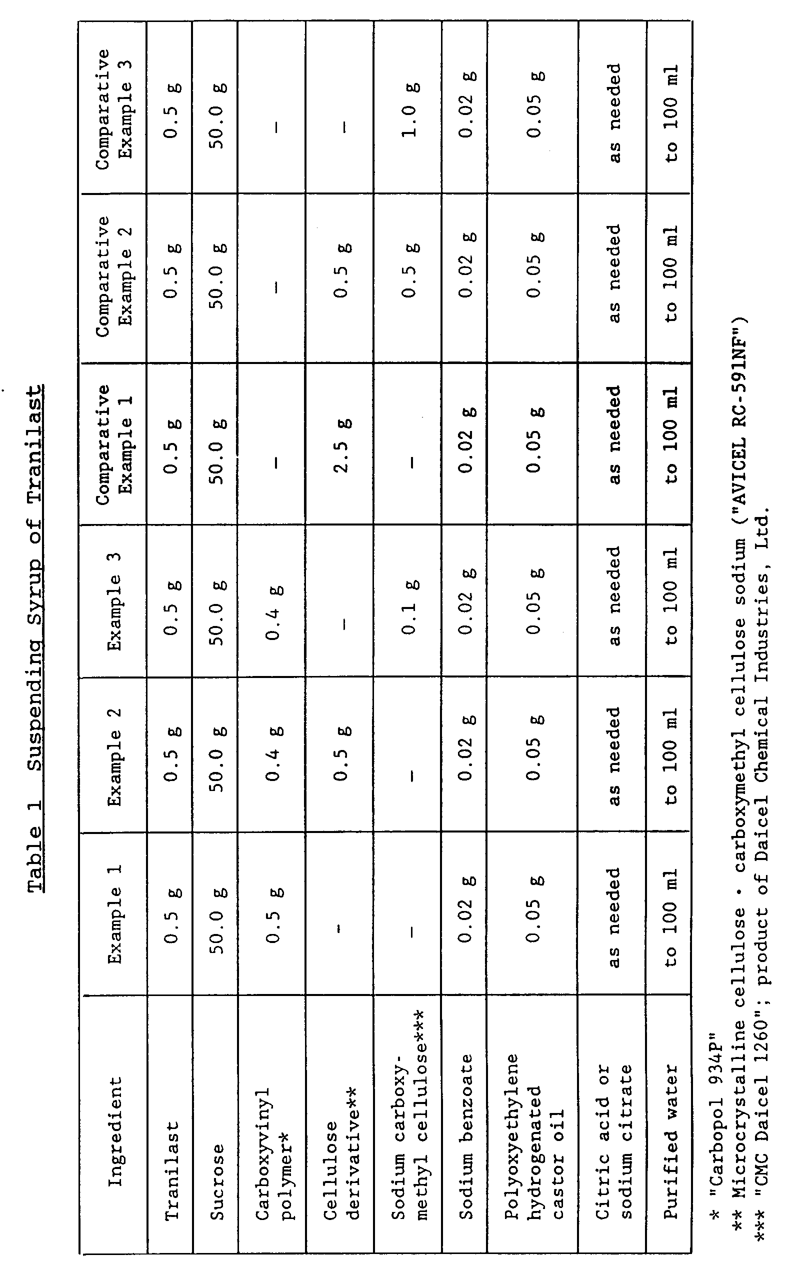

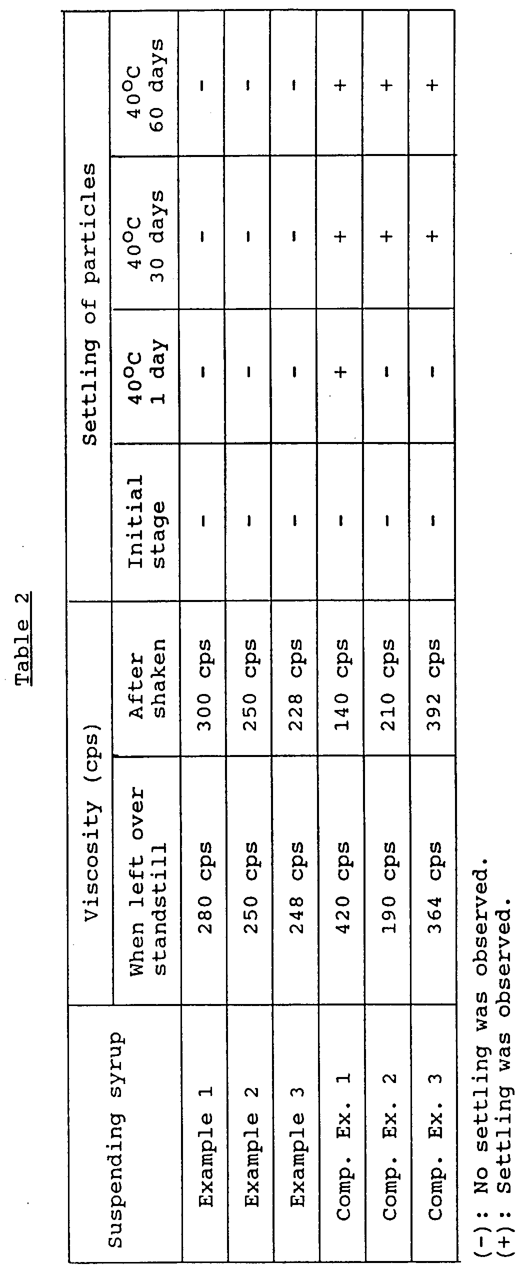

As is apparent from the above test results, it is understood that, compared to the suspending syrups of the comparative examples, those of the present invention underwent smaller viscosity variations even when shaken, remained free from settling on standing and were thermally stable.

### Examples 4-11

Following the procedures of Examples 1-3, suspending syrups of the following compositions were prepared.

### Suspending syrup of mefenamic acid (Example 4):

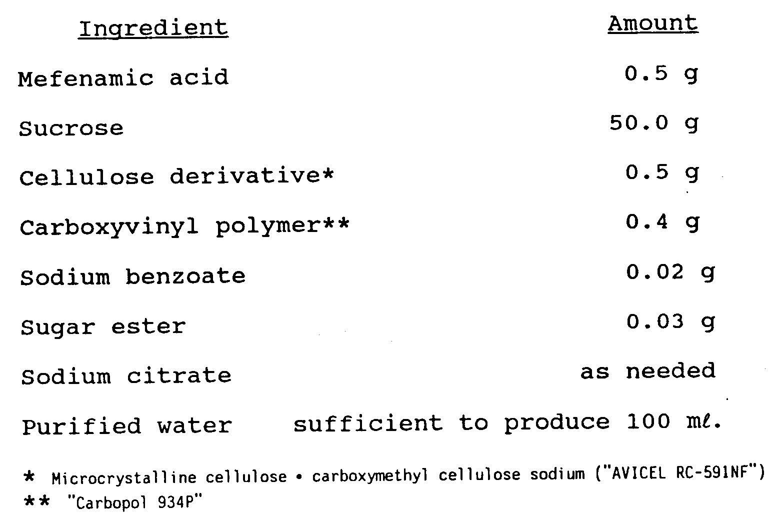

### Suspending syrup of fenbufen (Example 5):

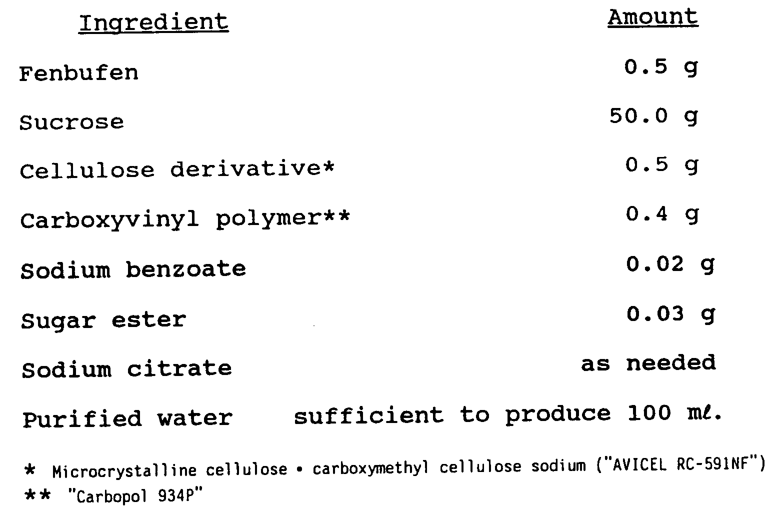

### Suspending syrup of tipepidine hibenzate (Example 6):

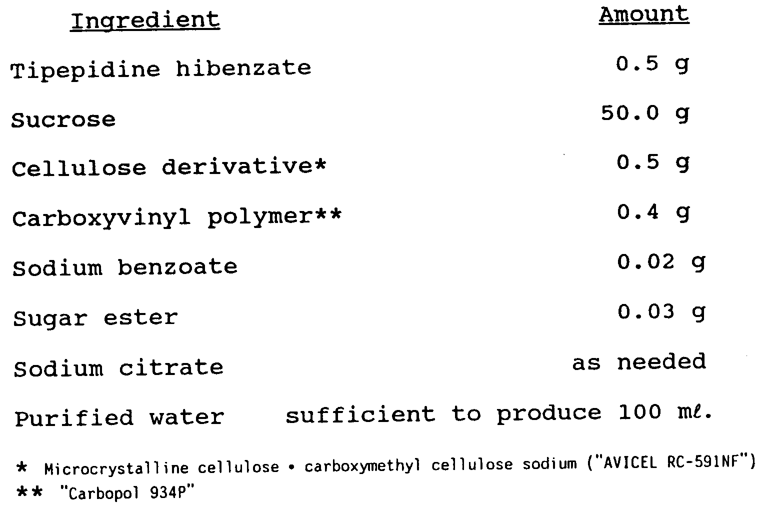

### Suspending syrup of pranoprofen (Example 7):

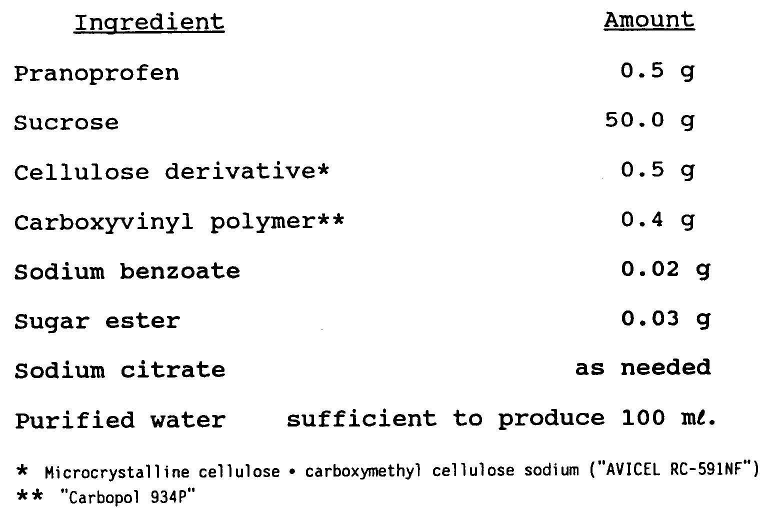

### Suspending syrup of erythromycin ethylsuccinate (Example 8):

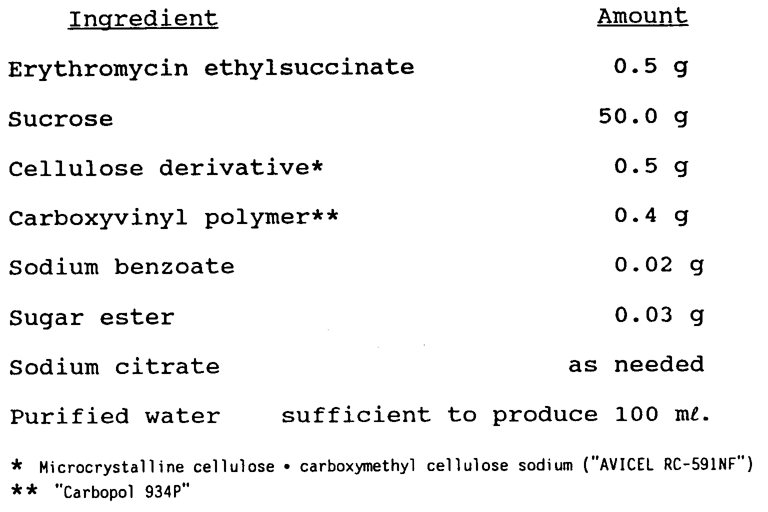

### Suspending syrup of domperidone (Example 9):

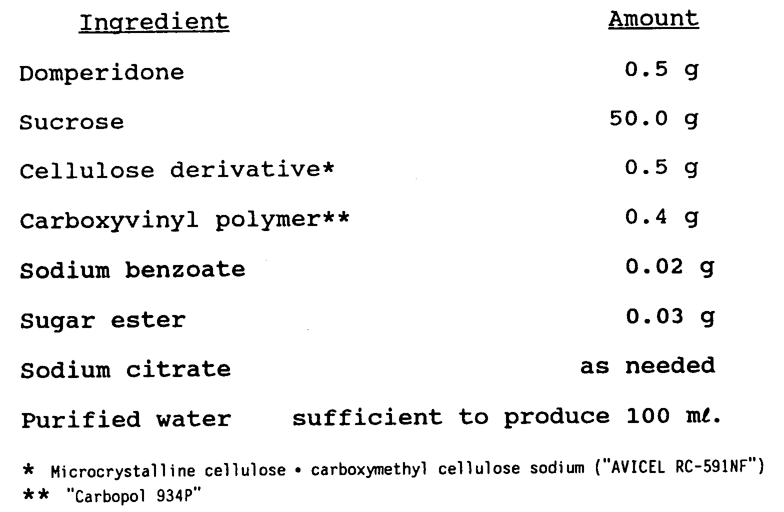

### Suspending syrup of tranilast (Example 10):

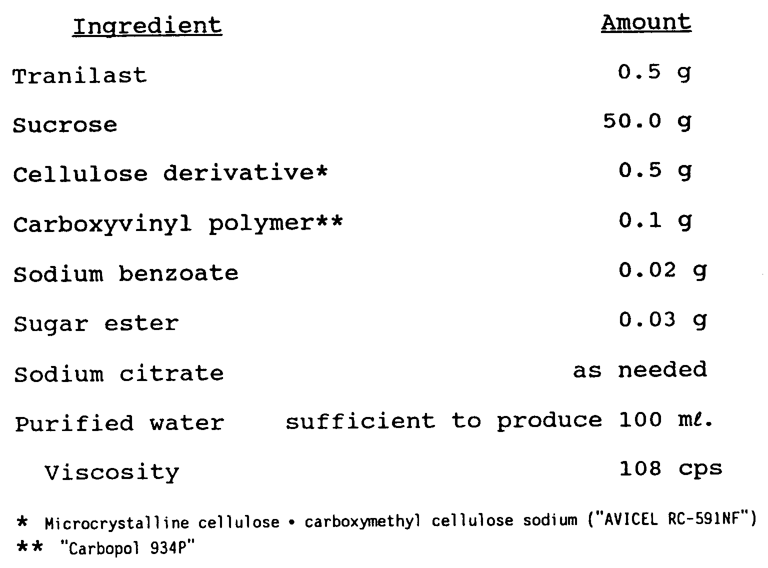

### Suspending syrup of tranilast (Example 11):

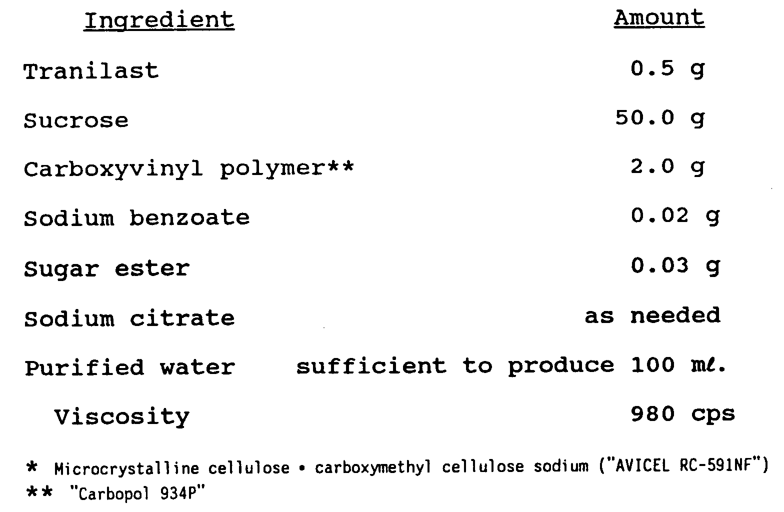

The resultant mixture of Example 11 was finally adjusted to pH 3.0 with sodium citrate.

## Claims

1. A suspending syrup with a slightly water-soluble medicine selected from the group consisting of tranilast, mefenamic acid, fenbufen, tipepidine hibenzate, pranoprofen, erythromycin ethyl succinate and domperidone, comprising carboxyvinyl polymer and having a viscosity of from 100 to 1,000 cps at 20°C.

2. The suspending syrup according to Claim 1, characterized in that the slightly water-soluble medicine is tranilast.

3. The suspending syrup of any one of Claims 1 or 2, wherein the content of carboxyvinyl polymer is 0.1-2.0 w/v.%.

## Patentansprüche

1. Suspensionsfähiger Sirup mit einem schwach wasserlöslichen Arzneimittel, ausgewählt aus der Gruppe bestehend aus Tranilast, Mefenaminsaure, Fenbufen, Tipepidinhibenzat, Pranoprofen, Erythromycinethylsuccinat und Domperidon, umfassen Carboxyvinylpolymer und eine Viskosität von 100 bis 1.000 cps bei 20°C aufweisend.

2. Suspensionsfähiger Sirup nach Anspruch 1, dadurch gekennzeichnet, daß das schwach wasserlösliche Arzneimittel Tranilast ist.

3. Suspensionsfähiger Sirup nach einem der Ansprüche 1 oder 2, worin der Gehalt an Carboxyvinylpolymer 0,1-2,0 Gew./Vol.-% beträgt.

## Revendications

1. Sirop de mise en suspension contenant un médicament légèrement hydrosoluble choisi dans le groupe constitué du tranilast, de l'acide méfénamique, du fenbufène, du o-(4-hydroxybenzoyl)benzoate de tipépidine, du pranoprofène, de l'éthylsuccinate d'érythromycine et de la dompéridone, comprenant un carboxyvinylpolymère et ayant une viscosité de 100 à 1 000 cps à 20 °C.

2. Sirop de mise en suspension selon la revendication 1, caractérisé en ce que le médicament légèrement hydrosoluble est le tranilast.

3. Sirop de mise en suspension selon l'une des revendications 1 et 2, dans lequel la teneur en carboxyvinylpolymère est de 0,1 à 2,0 % p/v.
